# EUROPÄISCHE PATENTSCHRIFT

(11) **EP 1 171 087 B1**
(45) Veröffentlichungstag und Bekanntmachung des Hinweises auf die Patenterteilung: **05.11.2003**
(21) Anmeldenummer: 00926860.8
(22) Anmeldetag: 11.04.2000
(51) Int. Cl.: A61K 7/38, A61K 7/32

(54) **DESODORIENDE ZUBEREITUNGEN ENTHALTEND WASSERLÖSLICHE -(1,3)-GLUCANE**
DEODORIZING PREPARATIONS COMPRISING WATER-SOLUBLE -(1,3)-GLUCANS
COMPOSITIONS DEODORANTES COMPRENANT DES -(1,3)-GLUCANES HYDROSOLUBLES

(30) Priorität: 20.04.1999 DE 19917743
(43) Veröffentlichungstag der Anmeldung: 16.01.2002
(73) Patentinhaber: Biotec Pharmacon ASA, 9008 Tromsö (NO)
(72) Erfinder: WACHTER, Rolf, D-40595 Düsseldorf (DE); GRIESBACH, Ute, D-40597 Düsseldorf (DE); FABRY, Bernd, D-41352 Korschenbroich (DE); ENGSTAD, Rolf, E., N-9008 Tromsö (NO)
(74) Vertreter: Melzer, Wolfgang, Dipl.-Ing.
(86) Internationale Anmeldenummer: EP0003192
(87) Internationale Veröffentlichungsnummer: WO00062752

(56) Entgegenhaltungen:
- WO-A-97/16164
- US-A- 3 659 025
- US-A- 4 012 333
- US-A- 5 653 967

## Beschreibung

### Gebiet der Erfindung

Die Erfindung befindet sich auf dem Gebiet der Kosmetik und betrifft desodorierende Zubereitungen mit einem Gehalt an speziellen Polysacchariden vom Typ der β-1,3-Glucane, Aluminiumchlorhydrat, Esteraseinhibitoren und/oder bakteriziden bzw. bakteriostatischen Wirkstoffen sowie die Verwendung der speziellen Glucane zur Herstellung von desodorierenden Zubereitungen.

### Stand der Technik

Im Bereich der Körperpflege werden Desodorantien zur Beseitigung störender Körpergerüche eingesetzt. Diese entstehen bei der bakteriellen Zersetzung des an sich geruchlosen Schweißes, insbesondere in den feuchtwarmen Achselhöhlen oder unter ähnlichen, den Mikroorganismen gute Lebensumstände bietenden Bedingungen. Körpergerüche können durch geeignete Riechstoffe überdeckt werden. Man kann sie auch bekämpfen, indem man Präparate einsetzt, welche die Schweißabsonderung selbst hemmen oder die Zersetzung des Schweißes inhibieren (sogenannte Antihidrotika, Antiperspirantien oder Antitranspirantien). Typische Beispiele für derartige Substanzen sind Aluminiumverbindungen wie Aluminiumsulfat oder Aluminiumchlorhydrat, Zinksalze und Citronensäureverbindungen. Eine Übersicht hierzu findet sich beispielsweise in **Umbach (Hrsg.), "Kosmetik", S.141f., Thieme Verlag, Stuttgart, 1988.**

Aus der täglichen Lebenserfahrung ist jedoch klar, daß das Problem der Geruchsinhibierung, insbesondere bei Hitze oder körperlicher Betätigung keineswegs vollständig gelöst ist. Die Produkte des Marktes vermögen weder die Absonderung von Schweiß noch die Bildung von Gerüchen dauerhaft zu unterbinden. Vielmehr ist die Inhibierung zeitlich begrenzt und auch davon abhängig, in welchem Umfang Schweiß abgesondert wird.

In diesem Zusammenhang sei auf die Patentschrift **US 5,223,491** hingewiesen, in der vorgeschlagen wird, ein carboxymethyliertes β-1,3-Glucan, das aus dem Hefepilz *Saccharomyces cerevisiae* extrahiert wurde, für die topische Anwendung einzusetzen. Das Glucan ist jedoch wasserunlöslich und kann daher nur mit großen Schwierigkeiten formuliert werden. Gemäß der Lehre der beiden Druckschriften **DE-A1 3744345** (Lomapharm) und **EP-B1 0175667** (Larm) eignen sich Glucane zur Stimulation der Makrophagenaktivität. Die pharmazeutische Wirkung verschiedener Glucane ist des weiteren aus den beiden Europäischen Patentanmeldungen **EP-A1 045338** (Debat) und **EP-A1 0561408** (Kaken) bekannt. Gegenstand der Europäischen Patentschrift **EP-B1 0500718** (Donzis) ist der Einsatz von wasserunlöslichen β-(1,3)-Glucanen, die aus den Zellwänden von Hefen gewonnen werden, zur Revitalisierung der Haut.

Die internationale Patentanmeldung **WO 97/16164** (Henkel) beschreibt die Verwendung von Aluminiumchlorhydrat, Esteraseinhibitoren (z.B. Triethylcitrat) und kationischen Biopolymeren (z.B. Chitosan) als Inhaltsstoffe zur Herstellung von desodorierenden Zusammensetzungen. Die kationischen Biopolymere dienen dabei als bakteriostatischer Wirkstoff.

Demzufolge besteht ein andauerndes Bedürfnis nach Produkten, die hinsichtlich der Minimierung der Schweißabsonderung und der Verminderung der Geruchsbildung verbessert sind und dabei gleichzeitig noch eine erhöhte hautkosmetische Verträglichkeit, d.h. ein vermindertes Irritationspotential gegenüber besonders empfindlicher Haut aufweisen. Die Aufgabe der Erfindung hat somit darin bestanden, derartige Produkte zur Verfügung zu stellen.

### Beschreibung der Erfindung

Gegenstand der Erfindung sind desodorierende Zubereitungen, enthaltend
(a) Aluminiumchlorhydrat,
(b) Esteraseinhibitoren und/oder
(c) bakterizide bzw. bakteriostatische Wirkstoffe, die dadurch gekennzeichnet sind, dass sie ferner wasserlösliche β-(1,3)-Glucane, im wesentlichen frei von β-(1,6)-Verknüpfungen, enthalten.

Ein weiterer Gegenstand der Erfindung ist die Verwendung von wasserlöslichen β-(1,3)-Glucanen, die im wesentlichen frei von β-(1,6)-Verknüpfungen sind, zur Herstellung von desodorierenden Zubereitungen.

Die Verwendung von Aluminiumchlorhydraten, Esteraseinhibitoren (z.B. Triethylcitrat) und bakteriziden Wirkstoffen (z.B. Chitosan) zur Herstellung von desodorierenden und/oder schweißhemmenden Zusammensetzungen ist aus dem Stand der Technik bekannt. Überraschenderweise wurde gefunden, daß die speziellen Glucane die Aktivität esterolytischer Enzyme bereits im unteren ppm-Bereich inhibieren und zusammen mit den vorgenannten Komponenten eine synergistische desodorierende Wirkung erzielt wird. Die Polysaccharide wirken selektiv auf Serinesterasen bzw. Serinproteasen ohne das biologische Gleichgewicht der Hautflora zu beeinträchtigen. Gleichzeitig führt der Einsatz der Glucane zu einer Immunstimulation und einer Verbesserung der hautkosmetischen Verträglichkeit der Produkte, so daß sie auch in kosmetischen Zubereitungen Verwendung finden.

### Wasserlösliche β-(1,3)-Glucane

Unter der Bezeichnung Glucane werden Homopolysaccharide auf Basis der Glucose verstanden. Je nach sterischer Verknüpfung unterscheidet man zwischen β-(1,3)-, β-(1,4)- und β-(1,6)-Glucanen. β-(1,3)-Glucane weisen meist eine helicale Struktur auf, während Glucane mit einer 1,4-Verknüpfung im allgemeinen eine lineare Struktur besitzen. Die β-Glucane der Erfindung besitzen eine (1,3)-Struktur, d.h. sie sind weitgehend frei von unerwünschten (1,6)-Verknüpfungen. Vorzugsweise werden solche β-(1,3)-Glucane eingesetzt, deren Seitenketten ausschließlich (1,3)-Verknüpfungen aufweisen. Insbesondere enthalten die Mittel Glucane, die auf Basis von Hefen der Familie *Saccharomyces,* speziell *Saccharomyces cerevisiae* erhalten werden. Glucane dieser Art sind in technischem Maße nach den Verfahren des Stands der Technik zugänglich. So beschreibt die Internationale Patentanmeldung WO **95/30022** (Biotec-Mackzymal) ein Verfahre zur Herstellung solcher Stoffe, bei dem man Glucane mit β-(1,3)- und β-(1,6)-Verknüpfungen in solcher Weise mit β-(1,6)-Glucanasen in Kontakt bringt, daß praktisch alle β-(1,6)-Verknüpfungen gelöst werden. Vorzugsweise werden zur Herstellung der Glucane Glucanasen auf Basis von *Trichodermia harzianum* eingesetzt. Soweit es die Herstellung und Zugänglichkeit der in den erfindungsgemäßen Mitteln enthaltenen Glucane angeht, wird in vollem Umfang auf die Offenbarung der oben genannten Schrift Bezug genommen. Die Glucane können in den Zubereitungen in Mengen von 0,1 bis 5, vorzugsweise 0,2 bis 5 und insbesondere 0,5 bis 1 Gew.-% - bezogen auf die Mittel - enthalten sein.

### Aluminiumchlorhydrat

Bei Aluminiumchlorhydraten der Komponente (a) handelt es sich um farblose, hygroskopische Kristalle, die an der Luft leicht zerfließen und beim Eindampfen wäßriger Aluminiumchloridlösungen anfallen. Aluminiumchlorhydrat wird zur Herstellung von schweißhemmenden und desodorierenden Zubereitungen eingesetzt und wirkt wahrscheinlich über ein Zusammenziehen bzw. Verkleben der Schweißdrüsen durch Eiweißfällung und/oder Feuchtigkeitsentzug [vgl. **J.Soc.Cosm.Chem. 24, 281 (1973)].** Unter der Marke Locron® der Hoechst AG, Frankfurt/FRG, befindet beispielsweise sich ein Aluminiumchlorhydrat im Handel, das der Formel [Al₂(OH)₅Cl]•2,5 H₂O entspricht und dessen Einsatz besonders bevorzugt ist [vgl. **J. Pharm.Pharmacol. 26, 531 (1975)].**

### Esteraseinhibitoren

Beim Vorhandensein von Schweiß im Achselbereich werden durch Bakterien extrazelluläre Enzyme - Esterasen, vorzugsweise Proteasen und/oder Lipasen - gebildet, die im Schweiß enthaltene Ester spalten und dadurch Geruchsstoffe freisetzen. Esteraseinhibitoren der Komponente b vorzugsweise Trialkylcitrate wie Trimethylcitrat, Tripropylcitrat, Tributylcitrat und insbesondere Triethylcitrat (Hydagen® CAT, Henkel KGaA, Düsseldorf/FRG) inhibieren die Enzymaktivität und reduzieren dadurch die Geruchsbildung. Wahrscheinlich wird dabei durch die Spaltung des Citronensäureesters die freie Säure freigesetzt, die den pH-Wert auf der Haut so weit absenkt, daß dadurch die Enzyme durch Acylierung inaktiviert werden. Weitere Stoffe, die als Esteraseinhibitoren in Betracht kommen, sind Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester.

### Bakterizide bzw. bakteriostatische Wirkstoffe

Bakterizide bzw. bakteriostatische Wirkstoffe der Komponente (c), welche die Keimflora beeinflussen und schweißzersetzende Bakterien abtöten bzw. in ihrem Wachstum hemmen, können ebenfalls in den Zubereitungen enthalten sein. Typische Beispiele sind insbesondere Chitosan und Phenoxyethanol. Besonders wirkungsvoll hat sich auch 5-Chlor-2-(2,4-dichlorphenoxy)-phenol erwiesen, das unter der Marke Irgasan® von der Ciba-Geigy, Basel/CH vertrieben wird.

### Gewerbliche Anwendbarkeit

Die speziellen β-1,3-Glucane haben sich für den beschriebenen Anwendungszweck enzyminhibierend erwiesen. Sie können daher insbesondere zur Herstellung von desodorierenden Zubereitungen verwendet werden, wobei sie alleine oder zusammen mit anderen Deowirkstoffen wie Aluminiumchlorhydraten, weiteren Esteraseinhibitoren und/oder bakteriziden bzw. bakteriostatischen Wirkstoffen eingesetzt werden. Die Zusammensetzungen können in einer bevorzugten Ausführungsform der Erfindung die Komponenten (a) bis (d) vorzugsweise in den folgenden Mengen - bezogen auf den Feststoffanteil - enthalten:
(a) 1,0 bis 50, vorzugsweise 10 bis 40 Gew.-% Aluminiumchlorhydrat,
(b) 0,01 bis 20, vorzugsweise 1,0 bis 5,0 Gew.-% Esteraseinhibitoren,
(c) 0,01 bis 5, vorzugsweise 0,1 bis 1,0 Gew.-% bakterizide bzw. bakteriostatische Wirkstoffe, und
(d) 0,01 bis 50, vorzugsweise 0,1 bis 10 Gew.-% wasserlösliche β-(1,3)-Glucane, die im wesentlichen frei von β-(1,6)-Verknüpfungen sind.

Hierbei besteht die Maßgabe, daß sich die Mengenangaben zu 100 Gew.-% ergänzen. Die Angaben verstehen sich jeweils auf den Aktivsubstanzgehalt der Komponenten.
Um die Wirkstoffe auf eine dosierbare, sparsame, bequeme und kosmetisch ansprechende Weise auf die Haut applizieren zu können, werden sie üblicherweise in Rezepturgrundlagen eingearbeitet. Als wichtigste Grundlagen sind zu nennen: Alkoholische und wäßrig/alkoholische Lösungen, Emulsionen, Gele, Öle, Wachs/Fett-Massen, Stiftpräparate und Puder. So können die erfindungsgemäßen Zubereitungen beispielsweise jeweils bis zu 60 Gew.-% niedere aliphatische Alkohole, vorzugsweise Ethanol sowie organische Säuren wie z.B. Glycolsäure enthalten. Weitere Einsatzstoffe sind Überfettungsmittel, Emulgatoren, Antioxidantien, Talkum, Kieselsäure (z.B. als Träger für das Aluminiumchlorhydrat), sowie Parfumöle, etherische Öle, Farbstoffe und - für Sprayanwendungen - Treibgase wie beispielsweise Propan und/oder Butan. Die Mittel kommen vorzugsweise als Roller (Roll-on-Emulsionen), Stifte, Deooder Pumpsprays in den Handel.

Die kosmetischen Zubereitungen können ferner als weitere Hilfs- und Zusatzstoffe milde Tenside, Ölkörper, Perlglanzwachse, Konsistenzgeber, Verdickungsmittel, Polymere, Siliconverbindungen, Fette, Wachse, Stabilisatoren, biogene Wirkstoffe, Antischuppenmittel, Filmbildner, Quellmittel, UV-Lichtschutzfaktoren, Hydrotrope, Konservierungsmittel, Insektenrepellentien, Selbstbräuner, Solubilisatoren, keimhemmende Mittel und dergleichen enthalten.

### Weitere Hilfs- und Zusatzstoffe

Typische Beispiele für geeignete milde, d.h. besonders hautverträgliche **Tenside** sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, Ethercarbonsäuren, Alkyloligoglucoside, Fettsäureglucamide, Alkylamidobetaine und/oder Proteinfettsäurekondensate, letztere vorzugsweise auf Basis von Weizenproteinen.

Als **Ölkörper** kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von Hydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, Guerbetcarbonate, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, Ringöffnungsprodukte von epoxidierten Fettsäureestern mit Polyolen, Siliconöle und/oder aliphatische bzw. naphthenische Kohlenwasserstoffe, wie z.B. wie Squalan, Squalen oder Dialkylcyclohexane in Betracht.

Als **Emulgatoren** kommen beispielsweise nichtionogene Tenside aus mindestens einer der folgenden Gruppen in Frage:
(1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(2) C_{12/18}-Fettsäuremono- und -diester von Anlagerungsprodukten von 1 bis 30 Mol Ethylenoxid an Glycerin;
(3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(4) Alkylmono- und -oligoglycoside mit 8 bis 22 Kohlenstoffatomen im Alkylrest und deren ethoxylierte Analoga;
(5) Anlagerungsprodukte von 15 bis 60 Mol Ethylenoxid an Ricinusöl und/oder gehärtetes Ricinusöl;
(6) Polyol- und insbesondere Polyglycerinester, wie z.B. Polyglycerinpolyricinoleat, Polyglycerinpoly-12-hydroxystearat oder Polyglycerindimeratisostearat. Ebenfalls geeignet sind Gemische von Verbindungen aus mehreren dieser Substanzklassen;
(7) Anlagerungsprodukte von 2 bis 15 Mol Ethylenoxid an Ricinusöl und/oder gehärtetes Ricinusöl;
(8) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose);
(9) Mono-, Di- und Trialkylphosphate sowie Mono-, Di- und/oder Tri-PEG-alkylphosphate und deren Salze;
(10) Wollwachsalkohole;
(11) Polysiloxan-Polyalkyl-Polyether-Copolymere bzw. entsprechende Derivate;
(12) Mischester aus Pentaerythrit, Fettsäuren, Citronensäure und Fettalkohol gemäß **DE 1165574 PS** und/oder Mischester von Fettsäuren mit 6 bis 22 Kohlenstoffatomen, Methylglucose und Polyolen, vorzugsweise Glycerin oder Polyglycerin;
(13) Polyalkylenglycole sowie
(14) Glycerincarbonat.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE 2024051 PS** als Rückfettungsmittel für kosmetische Zubereitungen bekannt.

C_{8/18}-Alkylmono- und -oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methylquaternierte Difettsäuretriethanolaminester-Salze, besonders bevorzugt sind.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als **Perlglanzwachse** kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Als **Konsistenzgebe**r kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten.

Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium hydroxypropyl hydrolyzed Collagen (Lamequat®L/Grünau), quaternierte Weizenpolypeptide, Polyethylenimin, kationische Siliconpolymere, wie z.B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, wie z.B. beschrieben in der **FR 2252840 A** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

**Als anionische, zwitterionische, amphotere und nichtionische Polymere** kommen beispielsweise Vinylacetat/Crotonsäure-Copolymere, Vinylpyrrolidon/Vinylacrylat-Copolymere, Vinylacetat/Butylmaleat/Isobomylacrylat-Copolymere, Methylvinylether/Maleinsäureanhydrid-Copolymere und deren Ester, unvemetzte und mit Polyolen vernetzte Polyacrylsäuren, Acrylamidopropyltrimethylammoniumchlorid/Acrylat-Copolymere, Octylacrylamid/Methylmethacrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/Dimethylaminoethylmethacrylat/Vinylcaprolactam-Terpolymere sowie gegebenenfalls derivatisierte Celluloseether und Silicone in Frage.

Geeignete **Siliconverbindungen** sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Eine detaillierte Übersicht über geeignete flüchtige Silicone findet sich zudem von Todd et al. in **Cosm. Toil. 91, 27 (1976).**

Typische Beispiele für **Fette** sind Glyceride, als **Wachse** kommen u.a. natürliche Wachse, wie z.B. Candelillawachs, Camaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage.

Als **Stabilisatoren** können Metallsalze von Fettsäuren, wie z.B. Magnesium-, Aluminium- und/oder Zinkstearat bzw. -ricinoleat eingesetzt werden.

Unter **biogenen Wirkstoffen** sind beispielsweise Tocopherol, Tocopherolacetat, Tocopherolpalmitat, Ascorbinsäure, Desoxyribonucleinsäure, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe zu verstehen.

Als **Antischuppenmittel** können Climbazol, Octopirox und Zinkpyrethion eingesetzt werden. Gebräuchliche **Filmbildner** sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

Als **Quellmittel** für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in **Cosm.Toil. 108, 95 (1993)** entnommen werden.

Unter **UV-Lichtschutzfaktoren** sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
- 3-Benzylidencampher bzw. 3-Benzylidennorcampher und dessen Derivate, z.B 3-(4-Methylbenzyliden)campher wie in der **EP 0693471 B1** beschrieben;
- 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
- Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
- Ester der Salicylsäure, vorzugsweise Salicylsäure-2-ethylhexylester, Salicylsäure-4-isopropylbenzylester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester;
- Triazinderivate, wie z.B. 2,4,6-Trianilino-(p-carbo-2'-ethyl-1'-hexyloxy)-1,3,5-triazin und Octyl Triazon, wie in der **EP 0818450** A1 beschrieben;
- Propan-1,3-dione, wie z.B. 1-(4-tert.Butylphenyl)-3-(4'methoxyphenyl)propan-1,3-dion;
- Ketotricyclo(5.2.1.0)decan-Derivate, wie in der **EP 0694521 B1** beschrieben.

Als wasserlösliche Substanzen kommen in Frage:
- 2-Phenylbenzimidazol-5-sulfonsäure und deren Alkali-, Erdalkali-, Ammonium-, Alkylammonium-, Alkanolammonium- und Glucammoniumsalze;
- Sulfonsäurederivate von Benzophenonen, vorzugsweise 2-Hydroxy-4-methoxybenzophenon-5-sulfonsäure und ihre Salze;
- Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol 1789), oder 1-Phenyl-3-(4'-isopropylphenyl)-propan-1,3-dion. Die UV-A und UV-B-Filter können selbstverständlich auch in Mischungen eingesetzt werden. Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. In Sonnenschutzmitteln werden bevorzugt sogenannte Mikro- oder Nanopigmente eingesetzt. Vorzugsweise wird mikronisiertes Zinkoxid verwendet.

Weitere geeignete UV-Lichtschutzfilter sind der Übersicht von P.Finkel in **SÖFW-Journal 122, 543 (1996)** zu entnehmen.

Neben den beiden vorgenannten Gruppen primärer Lichtschutzstoffe können auch sekundäre Lichtschutzmittel vom Typ der **Antioxidantien** eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl- , γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-Apalmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Zur Verbesserung des Fließverhaltens können ferner **Hydrotrope,** wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin.

Als **Konservierungsmittel** eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen.

Typische Beispiele für **keimhemmende Mittel** sind Konservierungsmittel mit spezifischer Wirkung gegen gram-positive Bakterien wie etwa 2,4,4'-Trichlor-2'-hydroxydiphenylether, Chlorhexidin (1,6-Di-(4-chlorphenyl-biguanido)-hexan) oder TCC (3,4,4'-Trichlorcarbanilid). Auch zahlreiche Riechstoffe und etherische Öle weisen antimikrobielle Eigenschaften auf. Typische Beispiele sind die Wirkstoffe Eugenol, Menthol und Thymol in Nelken-, Minz- und Thymianöl. Ein interessantes natürliches Deomittel ist der Terpenalkohol Farnesol (3,7,11-Trimethyl-2,6,10-dodecatrien-1-ol), der im Lindenblütenöl vorhanden ist und einen Maiglöckchengeruch hat. Auch Glycerinmonolaurat hat sich als Bakteriostatikum bewährt. Üblicherweise liegt der Anteil der zusätzlichen keimhemmenden Mittel bei etwa 0,1 bis 2 Gew.-% - bezogen auf den Feststoffanteil der Zubereitungen.

Als **Insekten-Repellentien** kommen N,N-Diethyl-m-touluamid, 1,2-Pentandiol oder Insekten-Repellent 3535 in Frage, als **Selbstbräuner** eignet sich Dihydroxyaceton.

Als **Parfümöle** seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Krautern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum, Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evemyl, Iraldein gamma, Phenylessigsäure, Geranylacetat, Benzylacetat, Rosenoxid, Romilllat, Irotyl und Floramat allein oder in Mischungen, eingesetzt.

Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

### Beispiele

Die Wirksamkeit der erfindungsgemäßen Mittel wurde stellvertretend über die Esteraseinhibierung bestimmt. Hierzu wurde nach 15 minütiger Einwirkzeit von 0,1 bis 5000 ppm der Testgemische auf die Esterase bei pH = 6 (Einstellung mit NaOH) deren Restaktivität parallel zu einer ungehemmten Kontrolle bestimmt (Standard = 100 %). Die Zusammensetzungen 1 bis 3 sind erfindungsgemäß, die Rezepturen V1 bis V4 dienen zum Vergleich. Tabelle 1 liefert eine Zusammenfassung der durchgeführten Beispiele (Mengenangaben in Gew.-%).

**Tabelle 1**

| **Rezepturen und Esteraseinhibierung** | | | | | | | |
|---|---|---|---|---|---|---|---|
| **Zusammensetzung/Performance** | **1** | **2** | **3** | **V1** | **V2** | **V3** | **V4** |
| Betaglucan* | 0,5 | 0,5 | 0,5 | - | - | - | - |
| Aluminiumchlorhydrat | - | 50,0 | 50,0 | 50,0 | - | 50,0 | 50,0 |
| Triethylcitrat | - | 5,0 | 5,0 | - | 5,0 | 5,0 | - |
| Chitosan | - | - | 1,0 | - | - | - | 5,0 |
| Ethanol | 20,0 | 20,0 | 20,0 | 20,0 | 20,0 | 20,0 | 20,0 |
| Wasser | ad 100 | | | | | | |
| ***Esterase Aktivität [%]*** | | | | | | | |
| *bei 2000 ppm* | - | - | - | 100 | 77 | 75 | 70 |
| *bei 500 ppm* | - | - | - | - | 100 | 100 | 100 |
| *bei 100 ppm* | 0 | 0 | 0 | - | - | - | - |
| *bei 10 ppm* | 5 | 0 | 0 | - | - | - | - |
| *bei 1 ppm* | 28 | 15 | 12 | - | - | - | - |
| *bei 0,1 ppm* | 72 | 66 | 55 | - | - | - | - |

| | | | | | | | |
|---|---|---|---|---|---|---|---|
| *) Highcareen® GS, Henkel KGaA, Düsseldorf/FRG | | | | | | | |

## Patentansprüche

1. Desodorierende Zubereitungen, enthaltend
(a) Aluminiumchlorhydrat
(b) Esteraseinhibitoren und/oder
(c) bakterizide bzw. bakteriostatische Wirkstoffe,
**dadurch gekennzeichnet,**
**dass** die Zubereitungen ferner
(d) wasserlösliche β-(1,3)-Glucane, im wesentlichen frei von β-(1,6)-Verknüpfungen, enthalten.

2. Zubereitungen nach Anspruch 1, **dadurch gekennzeichnet, daß** sie Glucane enthalten, die auf Basis von Hefen der Familie *Saccharomyces* erhalten werden.

3. Zubereitungen nach den Ansprüchen 1 und 2, **dadurch gekennzeichnet, daß** sie Glucane enthalten, die erhalten werden, indem man Glucane mit β-(1,3) und β-(1,6)-Verknüpfungen in solcher Weise mit β-(1,6)-Glucanasen in Kontakt bringt, daß praktisch alle β-(1,6)-Verknüpfungen gelöst werden.

4. Zubereitungen nach Anspruch 3, **dadurch gekennzeichnet, daß** sie Glucane enthalten, die zuvor mit Glucanasen auf Basis von *Trichodermia harzianum* behandelt worden sind.

5. Zubereitungen nach mindestens einem der Ansprüche 1 bis 4, **dadurch gekennzeichnet, daß** sie als Esteraseinhibitoren Trialkylcitrate enthalten.

6. Zubereitungen nach mindestens einem der Ansprüche 1 bis 5, **dadurch gekennzeichnet, daß** sie als bakterizide bzw. bakteriostatische Wirkstoffe Chitosane enthalten.

7. Zubereitungen nach mindestens einem der Ansprüche 1 bis 6, **dadurch gekennzeichnet, daß** sie - bezogen auf den Feststoffgehalt -
(a) 1,0 bis 50 Gew.-% Aluminiumchlorhydrat,
(b) 0,01 bis 20 Gew.-% Esteraseinhibitoren,
(c) 0,01 bis 5,0 Gew.-% bakterizide bzw. bakteriostatische Wirkstoffe und
(d) 0,01 bis 50 Gew.-% wasserlösliche β-(1,3)-Glucane, im wesentlichen frei von β-( 1,6)-Verknüpfungen,
enthalten, mit der Maßgabe, daß sich die Mengenangaben zu 100 Gew.-% ergänzen.

8. Verwendung von wasserlöslichen β-(1,3)-Glucanen, die im wesentlichen frei von β(1,6)-Verknüpfungen sind, zur Herstellung von desodorierenden Zubereitungen.

## Claims

1. Deodorizing preparations, containing
(a) aluminium chlorohydrate,
(b) esterase inhibitors, and/or
(c) bactericidal, or bacteriostatic, active ingredients, **characterized in that** the preparations also contain
(d) water-soluble β-(1,3) glucans, which are substantially free from β-(1,6) linkages.

2. Preparations according to Claim 1, **characterized in that** they contain glucans which are obtained on the basis of yeasts from the family *Saccharomyces.*

3. Preparations according to Claims 1 and 2, **characterized in that** they contain glucans which are obtained by contacting glucans having β-(1,3) and β-(1,6) linkages with β-(1,6) glucanases in such a way that practically all β-(1,6) linkages are broken.

4. Preparations according to Claim 3, **characterized in that** they contain glucans which had previously been treated with glucanases based on *Trichodermia harzianum*.

5. Preparations according to at least one of Claims 1 to 4, **characterized in that** they contain trialkyl citrates as esterase inhibitors.

6. Preparations according to at least one of Claims 1 to 5, **characterized in that** they contain chitosans as bactericidal, or bacteriostatic, active ingredients.

7. Preparations according to at least one of Claims 1 to 6, **characterized in that** they contain, based on the solids content,
(a) 1.0 to 50% by weight of aluminium chlorohydrate,
(b) 0.01 to 20% by weight of esterase inhibitors,
(c) 0.01 to 5.0% by weight of bactericidal, or bacteriostatic, active ingredients, and
(d) 0.01 to 50% by weight of water-soluble β-(1,3) glucans which are substantially free from β-(1,6) linkages,
with the proviso that the stated amounts sum to 100% by weight.

8. Use of water-soluble β-(1,3) glucans which are substantially free from β-(1,6) linkages for the preparation of deodorizing preparations.

## Revendications

1. Préparations désodorisantes, contenant :
a) du chlorhydrate d'aluminium,
b) des inhibiteurs de l'estérase et/ou
c) des principes actifs bactéricides ou bactériostatiques,
**caractérisées en ce que** les préparations contiennent en outre
d) des β-(1,3)-glucanes sensiblement exempts de liaisons β-(1,6).

2. Préparations selon la revendication 1, **caractérisées en ce qu'**elles contiennent des glucanes obtenus à partir de levures de la famille des *Saccharomyces*.

3. Préparations selon les revendications 1 et 2, **caractérisées en ce qu'**elles contiennent des glucanes qui sont obtenus par mise en contact de glucanes ayant des liaisons β-(1,3) et β-(1,6) avec des β-(1,6)-glucanases de telle façon que pratiquement toutes les liaisons β-(1,6) sont rompues.

4. Préparations selon la revendication 3, **caractérisées en ce qu'**elles contiennent des glucanes traités auparavant par des glucanases à base de *Trichodermia harzianum*.

5. Préparations selon au moins une des revendications 1 à 4, **caractérisées en ce qu'**elles contiennent des trialkylcitrates comme inhibiteurs de l'estérase.

6. Préparations selon au moins une des revendications 1 à 5, **caractérisées en ce qu'**elles contiennent des chitosans comme principes actifs bactéricides ou bactériostatiques.

7. Préparations selon au moins une des revendications 1 à 6, **caractérisées en ce qu'**elles contiennent, par rapport à la teneur en solide,
a) 1,0 à 50% en poids de chlorydrate d'aluminium,
b) 0,01 à 20% en poids d'inhibiteurs de l'estérase et/ou,
c) 0,01 à 5,0% en poids de principes actifs bactéricides ou bactériostatiques, et
d) 0,01 à 50% en poids de β-(1,3)-glucanes hydrosolubles, sensiblement exempts de liaisons β-(1,6)
à la condition que les indications quantitatives se complètent à 100% en poids.

8. Utilisation de β-(1,3)-glucanes hydrosolubles, sensiblement exempts de liaisons β-(1,6), pour la fabrication de préparations désodorisantes.
